# EUROPEAN PATENT SPECIFICATION

(11) **EP 1 037 627 B1**
(45) Date of publication and mention of the grant of the patent: **01.08.2001**
(21) Application number: 99928600.8
(22) Date of filing: 15.06.1999
(51) Int. Cl.: A61K 31/335

(54) **USE OF 11-(3-DIMETHYLAMINOPROPYLIDENE)-6,11-DIHYDRODIBENZ[B,E]OXEPIN-2-ACETIC ACID AND OF PHARMACEUTICALLY ACCEPTABLE SALTS THEREOF FOR THE MANUFACTURE OF A MEDICAMENT FOR THE TREATMENT OF NON-ALLERGIC OPHTHALMIC INFLAMMATORY DISORDERS AND FOR THE PREVENTION OF OCULAR NEOVASCULARIZATION**
VERWENDUNG VON 11-(3-DIMETHYLAMINOPROPYLIDEN)-6,11-DIHYDRODIBENZ[B,E]OXEPIN-2-ESSIGSÄURE UND DEREN PHARMAZEUTISCH ANNEHMBAREN SALZEN ZUR HERSTELLUNG EINES MEDIKAMENTS ZUR BEHANDLUNG NICHT-ALLERGISCH-BEDINGTEN AUGENENTZÜNDUNGEN UND ZUR PREVENTION DER OKULAREN NEOVASKULARISATION
UTILISATION D'ACIDE 11-(3-DIMETHYLAMINOPROPYLIDENE)-6,11-DIHYDRODIBENZ B,E]OXEPINE-2-ACETIQUE DANS LA PRODUCTION D'UN MEDICAMENT DE TRAITEMENT DE TROUBLES OPHTALMIQUES INFLAMMATOIRES NON ALLERGIQUES ET DE PREVENTION DE LA NEOFORMATION DE VAISSEAUX SANGUINS OCULAIRES

(30) Priority: 14.07.1998 US 92762 P
(43) Date of publication of application: 27.09.2000
(73) Proprietor: Alcon Laboratories, Inc., Fort Worth, Texas 76134-2099 (US)
(72) Inventor: YANNI, John, M., Burleson, TX 76028 (US); GAMACHE, Daniel, A., Arlington, TX 76017 (US); WEIMER, Lori, K., Arlington, TX 76017 (US)
(74) Representative: Keller, Günter, Dr.
(86) International application number: US9913275
(87) International publication number: WO0003705

(56) References cited:
- CUTARELLI, P.E. ET AL.: "The painful eye. External and Anterior Segment Causes" CLINICS IN GERIATRIC MEDICINE, vol. 15, no. 1, February 1999 (1999-02), pages 103-112, XP002119437
- YANNI, J.M. ET AL.: "Inhibition of Histamine-Induced Human Conjuctival Epithelial Cell Responses by Ocular Alergy Drugs" ARCH.OPHTHALM., vol. 117, no. 5, May 1999 (1999-05), pages 643-647, XP002119438 usa

## Description

### BACKGROUND OF THE INVENTION

### Field of the Invention

The present invention relates to the therapeutic and prophylactic use of 11-(3-dimethylamino-propylidene)-6,11-dihydrodibenz[b,e]oxepin-2-acetic acid for the manufacture of a medicament for the administration to the for treating and/or preventing cytokine release from human ocular cells and resulting ocular neovascularization or non-allergic inflammatory conditions.

### Description of the Related Art

As taught in U.S. Patent Nos. 4,871,865 and 4,923,892, both assigned to Burroughs Wellcome Co. ("the Burroughs Wellcome Patents"), certain carboxylic acid derivatives of doxepin, including 11-(3-dimethylaminopropylidene)-6,11-dihydrodibenz[b,e]oxepine-2-carboxylic acid and 11-(3-dimethylamino-propylidene)-6,11-dihydrodibenz[b,e]oxepine-2(E)-acrylic acid, have antihistaminic and antiasthmatic activity. These two patents classify the carboxylic acid derivatives of doxepin as mast cell stabilizers with antihistaminic action because they are believed to inhibit the release of autacoids (i.e., histamine, serotonin, and the like) from mast cells and to inhibit directly histamine's effects on target tissues. The Burroughs Wellcome Patents teach various pharmaceutical formulations containing the carboxylic acid derivatives of doxepin; Example 8 (I) in both of the patents discloses an ophthalmic solution formulation.

U.S. Patent 5,116,863, assigned to Kyowa Hakko Kogyo Co., Ltd., ("the Kyowa patent"), teaches that acetic acid derivatives of doxepin, including Z-11-(3-dimethylaminopropylidene)-6,11-dihydrodibenz[b,e]oxepin-2-acetic acid, have anti-allergic and anti-inflammatory activity. The anti-inflammatory activity is attributable to prostaglandin biosynthesis inhibiting activity (see Col. 28, lines 51-57). The doxepin derivatives disclosed by the Kyowa patent are represented by Compound (I): 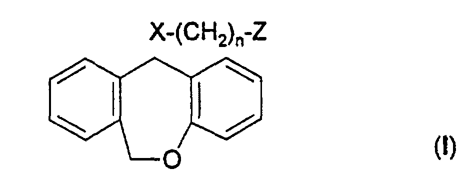 Compounds where X represents =N-, =CH- or -CH2- are described as having strong antiallergic activity, whereas compounds where X represents =N- are described as having strong antiinflammatory activity (see Col. 24, lines 20 - 57). Thus, for anti-inflammatory applications, the Kyowa patent suggests doxepin derivatives of Compound (I) where X is =N-.

The Kyowa patent demonstrates anti-allergic activity and anti-inflammatory activity in Wistar male rats. Medicament forms taught by the Kyowa patent for the acetic acid derivatives of doxepin include a wide range of acceptable carriers; however, only oral and injection administration forms are mentioned. In the treatment of allergic eye disease, such as allergic conjunctivitis, such administration methods require large doses of medicine.

U.S. Patent No. 5,641,805 discloses topical ophthalmic formulations containing 11-(3-dimethylamino-propylidene)-6,11-dihydrodibenz[b,e]oxepin-2-acetic acid for treating allergic eye diseases.

### Summary of the Inventior

The present invention provides the use of 11-(3-dimethylaminopropylidene)-6,11-dihydrodibenz [b,e] oxepin-2-acetic acid (referred to as "Compound A" hereinafter) or a pharmaceutically acceptable salt thereof for the manufacture of a medicament for administration to the eye for treating or preventing ophthalmic neovascularization and non-allergic inflammatory disorders involving cytokine release from human ocular cells. The formulation may contain the *cis* isomer of Compound A (Z-11-(3-dimethylaminopropylidene)-6,11-dihydrodibenz-[b,e]oxepin-2-acetic acid), the *trans* isomer of Compound A (E-11-(3-dimethylaminopropylidene)-6,11-dihydrodibenz[b,e]oxepin-2-acetic acid), or a combination of both the *cis* and the *trans* isomers of Compound A. Unless specified otherwise, "11-(3-dimethylaminopropylidene)-6,11-dihydrodibenz-[b,e]oxepin-2-acetic acid" or "Compound A" means the *cis* isomer, the *trans* isomer or a mixture of both. "*Cis* isomer" means the *cis* isomer substantially free of the *trans* isomer; *"trans* isomer" means the *trans* isomer substantially free of the *cis* isomer. One isomer is "substantially free" of the other isomer if less than about two percent of the unwanted isomer is present.

### Detailed Description of the Invention

Compound A is a known compound and both the *cis* and the *trans* isomers of Compound A can be obtained by the methods disclosed in U.S. Patent No. 5,116,863,

Examples of the pharmaceutically acceptable salts of Compound A include inorganic acid salts such as hydrochloride, hydrobromide, sulfate and phosphate; organic acid salts such as acetate, maleate, fumarate, tartrate and citrate; alkali metal salts such as sodium salt and potassium salt; alkaline earth metal salts such as magnesium salt and calcium salt; metal salts such as aluminum salt and zinc salt; and organic amine addition salts such as triethylamine addition salt (also known as tromethamine), morpholine addition salt and piperidine addition salt.

Compound A may be administered to the eye in a variety of ways. The most preferred way is by means of conventional topical ophthalmic formulations, such as solutions, suspensions or gels. Alternatively, Compound A may be administered to the eye via injection or implant. Depending upon the type of formulation, conventional ingredients will be combined with Compound A. The preferred formulation for topical ophthalmic administration of Compound A is a solution administered as eye drops. The preferred form of Compound A in the ophthalmic formulations of the present invention is the *cis* isomer. A general method of preparing an eye drop formulation of the present invention is described below as a nonlimiting example.

Compound A and an isotonic agent are added to sterilized purified water, and if required, a preservative, a buffering agent, a stabilizer, a viscous vehicle and the like are added to the solution and dissolved therein. The concentration of Compound A is 0.0001 to 5 w/v %, preferably 0.0001 to 0.001 w/v %, and most preferably 0.0005 w/v %, based on the sterilized purified water. After dissolution, the pH is adjusted with a pH controller to be within a range suitable for use as an ophthalmic medicine, preferably within the range of 4.5 to 8.

Sodium chloride, glycerin, mannitol or the like may be used as the isotonic agent; p-hydroxybenzoic acid ester, benzalkonium chloride or the like as the preservative; sodium hydrogenphosphate, sodium dihydrogenphosphate, boric acid or the like as the buffering agent; sodium edetate or the like as the stabilizer; polyvinyl alcohol, polyvinyl pyrrolidone, polyacrylic acid or the like as the viscous vehicle; and sodium hydroxide, hydrochloric acid or the like as the pH controller.

If required, other ophthalmic drugs such as epinephrine, naphazoline hydrochloride, berberine chloride, sodium azulenesulfonate, lysozyme chloride, glycyrrhizate and the like may be added.

The eye drops produced by the above method typically need only be applied to the eyes a few times a day in an amount of one to several drops at a time, though in more severe cases the drops may be applied several times a day. A typical drop is about 30 µl.

According to the use of the present invention, ophthalmic formulations containing Compound A are used to inhibit pro-inflammatory cytokine secretion from human ocular cells, such as human conjunctival epithelial cells. This type of cytokine secretion (e.g., IL-6 and IL-8) can stimulate ocular neovascularization (see, for example, Yoshida et al., IOVS, 39:1097 (1998)) and other non-allergic inflammatory conditions, such as dry eye, keratitits, blepharitis, uveitis and inflammation related to infection, for example.

Certain embodiments of the invention are illustrated in the following examples.

### Example 1: Preferred Topical Ophthalmic Solution Formulation

| Ingredient | Concentration (W/V%) |
|---|---|
| Compound A•HCl | 0.111* |
| Dibasic Sodium Phosphate (Anhydrous), USP | 0.5 |
| Sodium Chloride, USP | 0.65 |
| Benzalkonium Chloride | 0.01 |
| Sodium Hydroxide, NF 7.0 | q.s. pH = |
| Hydrochloric Acid, NF 7.0 | q.s. pH = |
| Purified Water | q.s. 100 |

| | |
|---|---|
| * 0.111% Compound A•HCl is equivalent to 0.1% Compound A | |

### Example 2: Topical Ophthalmic Gel Formulation

| Ingredient | Concentration (W/V%) |
|---|---|
| Compound A•HCl | 0.11* |
| Carbopol 974 P | 0.8 |
| Disodium EDTA | 0.01 |
| Polysorbate 80 | 0.05 |
| Benzalkonium Chloride, Solution | 0.01+5 xs |
| Sodium Hydroxide | q.s. pH 7.2 |
| Hydrochloric acid | q.s. pH 7.2 |
| Water for Injection | q.s. 100 |

| | |
|---|---|
| * 0.11% Compound A•HCl is equivalent to 0.1% Compound A | |

### Example 3: Inhibition of Cytokine Release

### A. Human Conjunctival Epithelial Cell (HCE) Cultures.

Methods detailing the preparation of primary epithelial cell cultures and cytokine release studies using these cells have been described. See Gamache, et al., "Secretion of proinflammatory cytokines by human conjunctival epithelial cells," *Ocul Immunol Inflamm*., 5:117-128 (1997). Briefly, cultures of human conjunctival epithelial cells were initiated from donor tissues obtained within eight hours post mortem by various eye banks. The tissues were enzymatically digested overnight. Epithelial cells were gently scraped from the tissue surface, dissociated into a single cell suspension, and cultured in keratinocyte growth medium (Clonetics® , San Diego, CA). Cells were used only through passage 6. Cultures were maintained in a preconfluent state to prevent differentiation. Cells were identified as epithelial by positive keratin staining.

### B. Cytokine Assays.

Several compounds with histamine H₁ antagonist activity were evaluated for their ability to inhibit secretion of cytokines (IL-6 and IL-8) from cultured human conjunctival epithelial cells in response to histamine stimulation. Cells were plated at 2x10⁴ cells/well and cultured overnight at 5% CO₂/37°C. The following day, fresh medium containing test compound was added directly to wells and the cells were incubated for 30 minutes prior to 24-hour stimulation with histamine (30 µM). Three separate culture wells were used for each treatment group. At harvest, supernatants were collected, centrifuged at 200 x g, and stored at -20°C. Samples were analyzed for IL-6 and IL-8 by ELISA (R&D Systems, Minneapolis, MN) as directed by the manufacturer. The sensitivities of each ELISA are as follows: IL-6 0.7 pg/ml and IL-8 3.0 pg/ml.

### C. Data Analysis

The antagonist potency (IC₅₀) was defined as the concentration of the drug required to produce 50% inhibition of the agonist-stimulated functional response. Data derived from the cytokine assays were calculated as mean and standard error (SEM) values which represent the variability among identically treated culture wells. The dose-dependent effect of pharmacological agents and IC₅₀'s were determined by linear regression. Data are expressed as mean ± S.E.M. from 3 - 5 independent experiments.

### D. Results.

Exposure of HCE to 30 *µ*M of histamine increased IL-6 and IL-8 secretion 1.59 + 0.19 and 1.80 + 0.28 fold above basal levels, respectively. (Basal levels of the cytokines were 153 ± 42 pg/ml, n = 4, for IL-6 and 197 ± 48 pg/ml, n = 6, for IL-8.) Treatment of HCE with drugs possessing anti-histaminic activity and available for topical ocular administration prior to histamine exposure resulted in concentration-dependent inhibition of IL-6 secretion and IL-8 secretion. The results are shown below in Table 1.

The potency of emedastine in intact cells is consistent with its activity determined in receptor binding assays using tissue homogenates. Levocabastine also inhibited the IL-6, and IL-8 secretion at a level consistent with its H₁-receptor binding affinity. Antazoline and pheniramine, two first generation topical ocular anti-histamine compounds, were dramatically less potent inhibitors of IL-6 and IL-8 secretion than predicted from their histamine H₁-receptor binding affinities (20 - 140-fold). Olopatadine, however, was more potent than predicted from its published histamine H₁-receptor binding affinity (36 nM). Olopatadine, antazoline and pheniramine exhibit similar H₁ binding affinities (32 - 39 nM). Yet, olopatadine was approximately 10-fold more potent as an inhibitor of cytokine secretion (IC₅₀'s of 5.5 nM and 1.7 nM for IL-6 and IL-8 secretion, respectively) than predicted from binding data. These results indicate that, unlike the other compounds tested, olopatadine's ability to inhibit cytokine secretion is attributable to something more than H,-receptor binding affinity.

**Table 1:**

| Histamine H₁ Antagonists: Inhibition of IL-6 and IL-8 Secretion in Human Conjunctival Epithelial Cells and H₁ Receptor Binding Affinities | | | |
|---|---|---|---|
| H₁ Antagonist | IL-6 IC₅₀ (nM) | IL-8 IC₅₀ (nM) | H₁ Binding Kᵢ (nM) |
| Emedastine^{a} | 2.5 | 4.0 | 1.22 * |
| Olopatadine^{b} | 5.5 | 1.7 | 36.0 § |
| Levocabastine^{c} | 25.1 | 11.9 | 52.6 * |
| Antazoline^{d} | 1014 | 652 | 38.4 * |
| Pheniramine^{e} | 4826 | 1216 | 33.9 * |

| | | | |
|---|---|---|---|
| ^{a} 1H-Benzimidazole,1-(2-ethoxyethyl)-2-(hexahydro-4-methyl-1H-1,4-diazepin-1-yl),(E)-2-butenedioate (1:2). | | | |
| ^{b} Z-11-(3-Dimethylaminopropylidene)-6,11-dihydrodibenz[b,e]oxepin-2-acetic acid. | | | |
| ^{c}(-)-traps-1-[cis-4-Cyano-4-(p-fluorophenyl)cyclohexyl]-3-methyl-4-phenylisonipecotic acid monohydrochloride. | | | |
| ^{d} 4,5-Dihydro-N-phenyl-N-(phenylmethyl)-1 H-imidazole-2-methanamine. | | | |
| ^{e} N,N-Dimethyl-γ-phenyl-2-pyridine-propanamine. | | | |
| * Sharif et al., *J Ocul Pharmacol*., 10:653-664 (1994) | | | |
| § Yanni et al., *Ann Allergy Asthma Immunol*., 79:541-545 (1997) | | | |

## Claims

1. Use of 11-(3-dimethylaminopropylidene)-6,11-dihydrodibenz[b,e]oxepin-2-acetic acid or a pharmaceutically acceptable salt thereof for the manufacture of a medicament for the administration to the eye for treating or preventing ocular neovascularization and non-allergic ophthalmic inflammatory disorders involving cytokine release from human ocular cells.

2. Use of Claim 1 wherein the medicament is a topically administrable solution and the amount of 11-(3-dimethylaminopropylidene)-6,11-dihydrodibenz[b,e]oxepin-2-acetic acid is from 0.0001 w/v.% to 5% (w/v).

3. Use of Claim 2 wherein the amount of 11-(3-dimethylaminopropylidene)-6,11-dihydrodibenz[b,e]oxepin-2-acetic acid is from 0.0001 to 0.001 % (w/v).

4. Use of Claim 3 wherein the amount of 11-(3-dimethylaminopropylidene)-6,11-dihydrodibenz[b,e]oxepin-2-acetic acid is 0.0005% (w/v).

5. Use of Claim 1 wherein the 11-(3-dimethylaminopropylidene)-6,11-dihydrodibenz[b,e]oxepin-2-acetic acid is (Z)-11 -(3-dimethylaminopropylidene)-6,11 -dihydrodibenz[b,e]oxepin-2-acetic acid, substantially free of (E)-11-(3-dimethylaminopropylidene)-6,11-dihydrodibenz[b,e]oxepin-2-acetic acid.

6. Use of Claim 5 wherein the composition is a topically administrable solution and the amount of (Z)-11-(3-dimethylaminopropylidene)-6,11-dihydrodibenz[b,e]oxepin-2-acetic acid is from 0.0001 to 5% (w/v).

7. Use of Claim 6 wherein the amount of (Z)-11-(3-dimethylaminopropylidene)-6,11-dihydrodibenz[b,e]oxepin-2-acetic acid is from 0.0001 to 0.001 % (w/v).

8. Use of Claim 7 wherein the amount of (Z)-11-(3-dimethylaminopropylidene)-6,11-dihydrodibenz[b,e]oxepin-2-acetic acid is 0.0005% (w/v).

9. Use of Claim 1 wherein the 11-(3-dimethylaminopropylidene)-6,11-dihydrodibenz[b,e]oxepin-2-acetic acid is (E)-11-(3-dimethylaminopropylidene)-6,11-dihydrodibenz[b,e]oxepin-2-acetic acid, substantially free of (Z)-11-(3-dimethylaminopropylidene)-6,11-dihydrodibenz[b,e]oxepin-2-acetic acid.

10. Use of Claim 9 wherein the composition is a topically administrable composition and the amount of (E)-11-(3-dimethylaminopropylidene)-6,11-dihydrodibenz[b,e]oxepin-2-acetic acid is from 0.0001 to 5% (w/v).

11. Use of Claim 10 wherein the amount of (E)-11-(3-dimethylaminopropylidene)-6,11-dihydrodibenz[b,e]oxepin-2-acetic acid is from 0.0001 to 0.001% (w/v).

12. Use of Claim 11 wherein the amount of (E)-11-(3-dimethylaminopropylidene)-6,11-dihydrodibenz[b,e]oxepin-2-acetic acid is 0.0005% (w/v).

13. Use of Claim 1 wherein the non-allergic ophthalmic inflammatory disorder is selected from the group consisting of dry eye, keratitits, blepharitis, uveitis and inflammation related to infection.

14. Use of Claim 1 wherein the ocular neovascularization and non-allergic ophthalmic inflammatory disorders involve cytokine release from human conjunctival epithelial cells.

## Patentansprüche

1. Verwendung von 11-(3-Dimethylaminopropyliden)-6,11-dihydrodibenz[b, e] oxepin-2-essisäure oder eines pharmazeutisch annehmbaren Salzes davon zur Herstellung eines Arzneimittels zum Auftragen auf das Auge zur Behandlung oder Verhütung einer Gefäßneubildung im Auge und von nichtallergischen ophthalmischen entzündlichen Störungen mit einer Cytokinfreisetzung aus menschlichen Augenzellen.

2. Verwendung nach Anspruch 1, wobei das Arzneimittel eine topisch verabreichbare Lösung ist und die Menge von 11-(3-Dimethylaminopropyliden)-6,11-dihydrodibenz[b,e]-oxepin-2-essigsäure 0,0001 % (G/V) bis 5% (G/V) ist.

3. Verwendung nach Anspruch 2, wobei die Menge an 11-(3-Dimethylaminopropyliden)-6,11-dihydrodibenz[b,e]oxepin-2-essigsäure 0,0001 bis 0,001% (G/V) ist.

4. Verwendung nach Anspruch 3, wobei die Menge an 11-(3-Dimethylaminopropyliden)-6,11-dihydrodibenz[b,e]oxepin-2-essigsäure 0,0005% (G/V) ist.

5. Verwendung nach Anspruch 1, wobei 11-(3-Dimethylaminopropyliden)-6,11-dihydrodibenz[b,e]oxepin-2-essigsäure (Z)-11-(3-Dimethylaminopropyliden)-6,11-dihydrodibenz[b,e]oxepin-2-essigsäure ist, die im Wesentlichen frei von (E)-11-(3-Dimethylaminopropyliden)-6,11-dihydrodibenz[b,e]oxepin-2-essigsäure ist.

6. Verwendung nach Anspruch 5, wobei die Zusammensetzung eine topisch verabreichbare Lösung ist und die Menge von (Z)-11-(3-Dimethylaminopropyliden)-6,11-dihydrodibenz-[b,e]oxepin-2-essigsäure 0,0001 bis 5% (G/V) ist.

7. Verwendung nach Anspruch 6, wobei die Menge von (Z)-11-(3-Dimethylaminopropyliden)-6,11-dihydrodibenz[b,e]-oxepin-2-essigsäure 0,0001 bis 0,001% (G/V) ist.

8. Verwendung nach Anspruch 7, wobei die Menge an (Z)-11-(3-Dimethylaminopropyliden)-6,11-dihydrodibenz[b,e]-oxepin-2-essigsäure 0,0005% (G/V) ist.

9. Verwendung nach Anspruch 1, wobei die 11-(3-Dimethylaminopropyliden)-6,11-dihydrodibenz[b,e]oxepin-2-essigsäure (E)-11-(3-Dimethylaminopropyliden)-6,11-dihydrodibenz[b,e]oxepin-2-essigsäure ist, die im Wesentlichen frei ist von (Z)-11-(3-Dimethylaminopropyliden)-6,11-dihydrodibenz[b,e]oxepin-2-essigsäure.

10. Verwendung nach Anspruch 9, wobei die Zusammensetzung eine topisch verabreichbare Zusammensetzung ist und die Menge an (E)-11-(3-Dimethylaminopropyliden)-6,11-dihydrodibenz[b,e]oxepin-2-essigsäure 0,0001 bis 5% (G/V) ist.

11. Verwendung nach Anspruch 10, wobei die Menge an (E)-11-(3-Dimethylaminopropyliden)-6,11-dihydrodibenz[b,e]-oxepin-2-essigsäure 0,0001 bis 0,001% (G/V) ist.

12. Verwendung nach Anspruch 11, wobei die Menge an (E)-11-(3-Dimethylaminopropyliden)-6,11-dihydrodibenz[b,e]-oxepin-2-essigsäure 0,0005% (G/V) ist.

13. Verwendung nach Anspruch 1, wobei die nicht allergische ophthalmische entzündliche Störung ausgewählt ist aus der Gruppe bestehend aus trockenem Auge, Keratitis, Blepharitis, Uveitis und einer mit einer Infektion in Beziehung stehenden Entzündung.

14. Verwendung nach Anspruch 1, wobei die Gefäßneubildung im Auge und die nicht allergischen ophthalmischen entzündlichen Störungen eine Cytokinfreisetzung aus menschlichen Bindehautepithelzellen beinhalten.

## Revendications

1. Utilisation d'acide 11-(3-diméthylaminopropylidène)-6,11-dihydrodibenz[b,e]oxépine-2-acétique ou d'un sel pharmaceutiquement acceptable de celui-ci pour la fabrication d'un médicament pour l'administration à l'oeil pour traiter ou prévenir une néovascularisation oculaire et des affections inflammatoires ophtalmiques non allergiques impliquant une libération de cytokines de cellules oculaires humaines.

2. Utilisation suivant la revendication 1, dans laquelle le médicament est une solution administrable topiquement et la quantité d'acide 11-(3-diméthylaminopropylidène)-6,11-dihydrodibenz[b,e]oxépine-2-acétique est de 0,0001% en poids/volume à 5% (poids/volume).

3. Utilisation suivant la revendication 2, dans laquelle la quantité d'acide 11-(3-diméthylaminopropylidène)-6,11-dihydrodibenz-[b,e]oxépine-2-acétique est de 0,0001 à 0,001% (poids/volume).

4. Utilisation suivant la revendication 3, dans laquelle la quantité d'acide 11-(3-diméthylaminopropylidène)-6,11-dihydrodibenz-[b,e]oxépine-2-acétique est de 0,0005% (poids/volume).

5. Utilisation suivant la revendication 1, dans laquelle l'acide 11-(3-diméthylaminopropylidéne)-6,11-dihydrodibenz[b,e]oxépine-2-acétique est de l'acide (Z)-11-(3-diméthylaminopropylidéne)-6,11-dihydrodibenz[b,e]oxépine-2-acétique, essentiellement exempt d'acide (E)-11-(3-diméthylaminopropylidène)-6,11-dihydrodibenz[b,e]oxépine-2-acétique.

6. Utilisation suivant la revendication 5, dans laquelle la composition est une solution administrable topiquement et la quantité d'acide (Z)-11-(3-diméthylaminopropylidène)-6,11-dihydrodibenz[b,e]oxépine-2-acétique est de 0,0001 à 5% (poids/volume).

7. Utilisation suivant la revendication 6, dans laquelle la quantité d'acide (Z)-11-(3-diméthylaminopropylidène)-6,11-dihydrodibenz[b,e]oxépine-2-acétique est de 0,0001 à 0,001% (poids/volume).

8. Utilisation suivant la revendication 7, dans laquelle la quantité d'acide (Z)-11-(3-diméthylaminopropylidène)-6,11-dihydrodibenz[b,e]oxépine-2-acétique est de 0,0005% (poids/volume).

9. Utilisation suivant la revendication 1, dans laquelle l'acide 11-(3-diméthylaminopropylidène)-6,11-dihydrodibenz[b,e]oxépine-2-acétique est de l'acide (E)-11-(3-diméthylaminopropylidène)-6,11-dihydrodibenz[b,e]oxépine-2-acétique, essentiellement exempt d'acide (Z)-11-(3-diméthylaminopropylidène)-6,11-dihydrodibenz[b,e]oxépine-2-acétique.

10. Utilisation suivant la revendication 9, dans laquelle la composition est une composition administrable topiquement et la quantité d'acide (E)-11-(3-diméthylaminopropylidène)-6,11-dihydrodibenz-[b,e]oxépine-2-acétique est de 0,0001 à 5% (poids/volume).

11. Utilisation suivant la revendication 10, dans laquelle la quantité d'acide (E)-11-(3-diméthylaminopropylidène)-6,11-dihydrodibenz[b,e]oxépine-2-acétique est de 0,0001 à 0,001% (poids/volume).

12. Utilisation suivant la revendication 11, dans laquelle la quantité d'acide (E)-11-(3-diméthylaminopropylidène)-6,11-dihydrodibenz[b,e]oxépine-2-acétique est de 0,0005% (poids/volume).

13. Utilisation suivant la revendication 1, dans laquelle l'affection inflammatoire ophtalmique non allergique est choisie dans le groupe comprenant l'oeil sec, les kératites, la blépharite, l'uvéite et les inflammations liées à une infection.

14. Utilisation suivant la revendication 1, dans laquelle le néovascularisation oculaire et les infections inflammatoires ophtalmiques non allergiques impliquent une libération de cytokines de cellules épithéliales conjonctivales humaines.
